# EUROPEAN PATENT APPLICATION

(11) **EP 4 721 731 A1**
(43) Date of publication of application: **08.04.2026**
(21) Application number: 24214071.3
(22) Date of filing: 20.11.2024
(51) Int. Cl.: A61K 9/20, A61K 31/4453, A61P 21/00, A61P 21/02

(54) **SUSTAINED RELEASE ORAL MEDICINAL PRODUCT CONTAINING TOLPERISONE HYDROCHLORIDE, ITS PRODUCTION AND USE**

(30) Priority: 02.10.2024 HU 2400448
(71) Applicant: MEDITOP Gyógyszeripari Kft., 2097 Pilisborosjenö (HU)
(72) Inventor: Fekete, Pál, 1141 Budapest (HU); Nacsa, Fruzsina, 1157 Budapest (HU); Gál, Lívia, 2000 Szentendre (HU); Öszi, Zsolt, 2220 Vecsés (HU)
(74) Representative: Kovári, Zoltán

(57) **Abstract**

The invention relates to sustained release oral medicinal product containing tolperisone, that contains tolperisone hydrochloride, betaine hydrochloride and a hydrophilic matrix-forming material and which medicinal product releases at least 80% of the tolperisone hydrochloride active ingredient within 6 hours. Furthermore, the invention relates to the manufacture of the above medicinal product and to its use.

## Description

### The field of the invention

The invention relates to a sustained release medicinal product containing tolperisone hydrochloride, a method for the production of such medicinal product and to the use of the medicinal product.

### The state of the art

Within the scope of the present invention, we are dealing with a sustained release tablet containing tolperisone hydrochloride (see below for the chemical structure). Tolperisone hydrochloride is a centrally acting muscle relaxant, the purpose of the use of which is the treatment of symptoms related to spastic paralysis, the reduction of painful muscle spasms, and the improvement of muscle tone and thus patient mobility. Due to the relatively low frequency of occurrence of sedation compared to other antispasmodic medications the tolperisone active ingredient has become a first-choice drug in antispasmodic therapy.

The chemical structure of tolperisone hydrochloride is as follows:

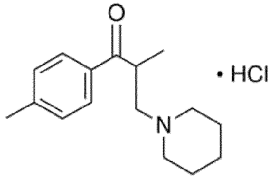

The chemical name of tolperisone is as follows: 2-methyl-1-(4-methylphenyl)-3-(1-piperidinyl)-1-propanone. After oral administration the tolperisone active ingredient is absorbed well from the small intestine, its peak concentration (Cₘₐₓ) is achieved after 0.5 to 1 hour, however bioavailability is only about 20% due to its significant first-pass metabolism. It is known that medicines absorbed from the digestive tract reach the liver via the vena portae, some are then extensively metabolised instantly, which is known as first pass effect in English.

The half-life of tolperisone is 3 to 4 hours, due to this the usual dose in the case of immediate active ingredient release products is three tablets a day, commonly 3 x 150 mg. In order to compensate for the short half-life, multiple developments have been performed in order to produce a medicinal product containing tolperisone that is suitable for once a day administration.

Such medicinal products suitable for administration once a day are known in the literature as sustained effect, sustained release, sustained active ingredient release, controlled release or retard products. In the present specification hereinafter, we use the term sustained release.

From the point of view of pharmaceutical formulation, the disadvantageous property of the tolperisone active ingredient is pH instability, as the piperidine ring opens easily in an environment at a pH value of over 6 resulting in various degradation products. This may present a problem both from the point of view of storage and release in the contents of the intestines, as such degradation of tolperisone may also occur within the pharmaceutical form during the several hours of release. In order to prevent the opening of the piperidine ring medicinal products containing tolperisone also include acidic excipients. Numerous acidic excipients have been described for this purpose, however, in practice citric acid is used for this purpose almost without exception.

Several solutions are known of according to the state of the art the subject of which is a sustained release medicinal product containing tolperisone.

The Hungarian patent application number P0200669 of Sanochemia Pharmazeutika Ag relates to a sustained release, orally administered medicinal product containing tolperisone that contains tolperisone or its salt as active ingredient in the form of a racemic mixture. The medicinal product is produced as a solid or liquid medicine in such a way that
(a) the crystalline, racemic tolperisone is coated in an airflow-operated coating column with a mixture of methanol and a chloroform solution of a synthetic, semi-synthetic or natural hydrogel; or
(b) microcapsules containing tolperisone are suspended in an aqueous solution saturated with tolperisone; or
(c) a synthetic, semisynthetic or natural hydrogel is granulated after the addition of water, the granulate obtained in this way, and serving as a binder, is dried then mixed with tolperisone and in the meantime diluted with an ethanol solution of a synthetic, semisynthetic or natural hydrogel, following this the mass is granulated, then after mixing the granulate with tabletting excipients it is pressed into tablets; or
(d) tolperisone is granulated in a granulating apparatus while adding an aqueous solution containing lactose and a synthetic, semisynthetic or natural hydrogel, the granulate obtained is made to pass through a sieve with magnesium stearate and finely divided silicon dioxide, then homogenised in a mixing apparatus, then the homogenate obtained is pressed into tablets.

Patent application number US20050196451A1 describes a controlled active ingredient release medicinal product in which tolperisone is formulated with a methacrylate-based polymer, such as with Eudragit^{®}. This pharmaceutically acceptable methacrylate-based polymer consists of polymers and/or copolymers containing a group of acrylic and/or methacrylic acid esters. Combinations of methacrylic acid polymers are used in patent application number US20050196451A1, then an organic solvent solution of these is used to produce tolperisone granulates, which are provided with an additional coating to slow down or delay the release of the active ingredient. This method is very complex and costly due to the separate granulation and coating processes.

Sanochemia Pharmazeutika Ag is also the holder of patent applications number WO2005094825 and WO2005084676, and Eudragit-type polymers are again used during the methods disclosed in these documents as the coating material. However, the granulation process implemented with an organic solvent pollutes the environment, involves the risk of explosion, is harmful to health, and causes an organic contaminant residue in the medicinal product.

Due to the above, the elaboration and use of production technologies that do not use organic solvents is of great significance in the production of medicinal products.

On the basis of pharmacokinetic data tolperisone is only absorbed from the upper section of the small intestine, therefore Sanochemia Pharmazeutika Ag developed medicinal products that remain in the stomach for a longer period of time as a result of gas generation or their hydrodynamic or geometrical properties.

Patent application number EP2228056A2 contains a description of a hydrodynamic tablet, i.e. a tablet with a lower density than that of the stomach content.

Hungarian patent registration number HU208920 discloses a solid retard pharmaceutical form provided with a coating that, in the case of oral administration, remains in the stomach and continuously releases the active ingredient there, while the stomach emptying process takes place periodically. Parts of the pharmaceutical form in question include a hydrophilic swelling component and the excipient sodium hydrogen carbonate, which produces carbon dioxide, which are surrounded by a membrane (polyvinyl-alcohol (PVA), polyethylene glycol(PEG) - fatty acid ester). The gas generation caused by the sodium hydrogen carbonate, on the one hand, obviously depends on the pH value of the stomach, therefore the active ingredient release of the pharmaceutical form may fluctuate, on the other hand, the carbon dioxide released in the stomach has physiological disadvantages.

European patent number EP2175839B1 relates to a controlled active ingredient release medicinal product containing tolperisone hydrochloride, which consists of several granulate cores, which are produced with a natural anionic polymer and a lipophilic excipient, using two-step granulation, which is then tabletted after the addition of a hydrophilic matrix-forming polymer. At least 50% of the active ingredient is released from the medicinal product in 8 hours following administration. The use of citric acid is recommended for improving the stability of the tolperisone active ingredient. Due to the two-step granulation process, the production method of the medicinal product is complex and costly.

It should be noted that none of the sustained release tolperisone products according to the patent applications and specifications presented above have been placed on the market.

MEDITOP Gyógyszeripari Kft. is present on the market with an immediate-release film-coated tablet containing 50 and 150 mg tolperisone hydrochloride, and the usual daily dose of these medicinal products is one film-coated tablet three times a day.

On the basis of all this there is a need for a pharmaceutical form containing tolperisone hydrochloride that is a sustained release product and that overcomes the disadvantages of the solutions presented above and, at the same time, is adapted for the control of the release of the tolperisone active ingredient and for reducing the frequency of administration. Therefore, the objective was set to create a pharmaceutical form containing tolperisone hydrochloride that ensures sustained release of the active ingredient and, due to this, the frequency of administration is lower than in the case of immediate release medicinal products, and with which, in spite of the less frequent administration, an even blood concentration of the active ingredient may be ensured. In order to realise this, we found a medicinal product for administration of the active ingredient twice a day to be suitable, as the morning and evening taking of medication is acceptable for patients, and on the basis of the absorption/metabolization properties of tolperisone, an even blood concentration may be ensured more simply.

According to the state of the art no medicinal product containing tolperisone is known of in which the sustained release of the tolperisone hydrochloride is realised in such a way that twice daily administration of the medicinal product is sufficient, thereby resulting in better patient cooperation and a medicinal product that can be produced in a simpler way than to date solutions and at a lower cost.

In other words, an important advantage of the solution according to the present invention, compared to the currently marketed products that are administered three times a day, is the provision of twice daily administration (morning and evening), furthermore, the production of the product is simpler, and thereby cheaper than the solutions described for the production of sustained release products.

### A brief presentation of the figures

Figure 1 illustrates the *in vitro* release data of the tablets originating from the production batches 1/C and 1/D included in example 2.
Figure 2 illustrates the *in vitro* release data of the production batches 2/A to 2/D included in example 3.
Figure 3 shows the pH-dependence of the composition included in example 4.

### A brief description of the invention

While searching for a composition of a sustained release medicinal product containing tolperisone suitable for industrial production, in a way surprising for a person skilled in the art, it was found that through the use of tolperisone in the form of a hydrochloride salt and with the appropriate selection of the excipients, a medicinal product with appropriate release properties may be produced using a simpler method than those known of to date. Medicinal products containing tolperisone known of to date must either be administered at least three times a day due to the insufficient bioavailability of the active ingredient or if the product is a single-dose per day, sustained release tolperisone product, its production is complex and therefore time-consuming and costly.

The present invention relates to the production of a product containing tolperisone hydrochloride, 80% of the active ingredient of which is released in 6 hours (measured using a dissolution test performed by a paddle method). As in this case, in the case of medication administration twice daily, with an interval of approx. 12 hours, the therapeutic level of tolperisone may be ensured. In the case of sustained release products, betaine hydrochloride is used in the composition to create the acidic micro-environment in order to ensure the stability of the tolperisone hydrochloride during release after administration.

During the development of the sustained release pharmaceutical form of the tolperisone product for twice daily administration according to the present invention, it was surprising to find that in order to produce the medicinal product it was sufficient to use 5 to 15 mass% hydrophilic matrix-forming polymer with respect to the mass of the product, furthermore that the addition of an amount of betaine hydrochloride excipient at a ratio of 1:10 - 1:5 with respect to the amount of tolperisone hydrochloride does not influence the hardness of the product nor the release speed of the active ingredient.

Therefore, in accordance with the above, the present invention relates to a sustained release oral medicinal product containing tolperisone that contains tolperisone hydrochloride as active ingredient, a hydrophilic matrix-forming material and betaine hydrochloride as excipient ensuring an acidic medium, and from which medicinal product at least 80% of the tolperisone hydrochloride active ingredient content is released in 6 hours (measured using a dissolution test performed by paddle method).

According to a preferable embodiment of the present invention the sustained release oral medicinal product containing tolperisone also contains a lubricant and an antiadhesion excipient.

According to a preferable embodiment of the sustained release oral medicinal product containing tolperisone according to the present invention, it contains 60 to 80 mass% tolperisone hydrochloride, 5 to 15 mass% hydrophilic matrix-forming material, 5 to 15 mass% betaine hydrochloride, and optionally 3 to 6 mass% lubricant, and 1 to 3 mass% antiadhesion excipient with respect to the mass of the medicinal product.

According to an even more preferable embodiment of the sustained release oral medicinal product containing tolperisone according to the present invention, it contains 60 to 70 mass% tolperisone hydrochloride, 10 to 15 mass% hydrophilic matrix-forming material, 10 to 15 mass% betaine hydrochloride, and optionally 4 to 5 mass% lubricant, and 1 to 2 mass% antiadhesion excipient with respect to the mass of the medicinal product.

According to another preferable embodiment of the invention, the hydrophilic matrix-forming material is selected from the following list: hydroxypropyl methylcellulose (hypromellose), hydroxypropyl cellulose, polyethylene oxide, xanthan gum or polyacrylic acid; the hydrophilic matrix-forming material is more preferably hypromellose.

According to another preferable embodiment of the invention, the viscosity of the hydrophilic matrix-forming material (hypromellose) in a 2% aqueous solution is over 4000 centipoise (cP), more preferably nominally 100000 cP. According to the prescription of the United States Pharmacopeia the viscosity of such hypromellose measured using a Brookfield rotational viscometer may be 75% to 140% of the nominal value.

According to another preferable embodiment of the invention, the lubricant is selected from the following list: stearic acid, stearic acid salts, glycerine stearates; the lubricant is preferably stearic acid.

According to another preferable embodiment of the invention, the antiadhesion excipient is talc or silicon dioxide; more preferably talc.

In accordance with the above, according to another preferable embodiment of the present invention, the sustained release oral medicinal product containing tolperisone consists of tolperisone hydrochloride, hydrophilic matrix-forming material, betaine hydrochloride, talc and stearic acid.

The present invention also relates to a sustained release oral medicinal product containing tolperisone the pharmaceutical form of which is a tablet.

The sustained release oral medicinal product containing tolperisone according to the present invention contains 200 to 400 mg tolperisone hydrochloride, preferably 225 to 300 mg tolperisone hydrochloride.

The present invention also relates to a method for the production of the sustained release oral medicinal product containing tolperisone according to the above, which method contains the following steps:
a) all of the tolperisone hydrochloride, all of the betaine hydrochloride and at least 30% of the hydrophilic matrix-forming material or all of it are homogenised, then granulated with the addition of water;
b) the granulate obtained in step a) is dried;
c) the dry granulate is sieved;
d) the granulate according to step c) is homogenised with the lubricant, the antiadhesion excipient and, optionally, with the remainder of the hydrophilic matrix-forming material;
e) the homogenised material obtained from step d) is pressed into tablets;
f) the tablets according to step e) are optionally provided with a film coating.

Furthermore, the present invention relates to the use of the sustained release oral medicinal product containing tolperisone according to the above for the treatment of symptoms related to spastic paralysis, or for the reduction of painful muscle spasms, or the use in a method for the improvement of muscle tone, where the form of the medicinal product is preferably a tablet.

Furthermore, the present invention also relates to the use of the sustained release oral medicinal product containing tolperisone according to the above for the treatment of symptoms related to spastic paralysis, or for the reduction of painful muscle spasms, or the use in a method for the improvement of muscle tone, where the medicinal product is administered to the patient twice a day.

### A detailed description of the invention

If not mentioned to the contrary, % in the present specification is understood to mean mass percent (mass%), and ratios are understood to mean the mass ratios of the individual components.

In the scope of the present specification if a numerical value is given, this is understood to mean that the last digit of the given number shows the precision of the given value according to the rounding-up rules. Therefore, for example, 80% is understood to mean all values falling within the range of 79.5% to 80.4%.

In the scope of the present specification, if a range between two values is defined, then the concrete numerical values indicated as the limit values of the range are viewed as constituting parts of the range.

Within the scope of the present invention sustained release oral medicinal product containing tolperisone is understood to mean a medicinal product that contains tolperisone hydrochloride as active ingredient, hydrophilic matrix-forming material and betaine hydrochloride as excipient ensuring an acidic medium, furthermore, from which at least 80% of the tolperisone hydrochloride active ingredient is released in 6 hours after administration, in other words, instead of the administration three times a day in the case of the tolperisone products currently marketed, it is sufficient to administer it twice daily. The measurement of the active ingredient release of the medicinal product is performed by paddle apparatus and method according to the prescriptions of the monograph *Ph*. *Eur. 2.9.3. Dissolution test for solid dosage forms.* A total of 900 ml 37 ± 0.5 °C, 0.1 N hydrochloric acid solution was used as dissolution medium and a pH 4.5, and a pH 6.8 buffer was used, the mixing speed was 50 revolutions/minute. The amount of active ingredient released from the tablets was given as a percentage of the nominal active ingredient content of the tablets. The tests were performed using an Erweka automatic tablet dissolution tester apparatus. The results are given as the average of the results measured after the given mixing (dissolution) time of six tablets.

After administration the sustained release products reach the stomach and then the intestinal system, in other words sustained release means that the medicinal product remains in the digestive system for a longer period of time while its active ingredient content is being continuously released. In the case of the present invention, in the case of twice a day, approx. every 12 hours administration, the therapeutic blood concentration of the tolperisone active ingredient may be ensured. Compared to sustained release medicinal products, immediate release medicinal products have numerous disadvantages, such as, for example, the fast metabolization and elimination of the active ingredient, which, in order to achieve systemic effects, demands that the dose be administered repeatedly at least three times a day, as in the case of the tolperisone active ingredient, for example. It is obvious for a person skilled in the art that leaving out the dose of medicine due during the day in the case of three times daily dosing reduces the efficacy of any product. A reason for leaving out the medicine dose due to be taken during the day may be forgetfulness, as on one hand the day-to-day tasks of a person may distract the patient's attention away the medication, on the other hand the majority of people that take medication are not usually at home during the day, and in such cases it may happen that the reason for leaving out the dose is that the patient does not have the medicinal product that they should be taking with him/her. 80% of the active ingredient is released from immediate release oral medicinal products containing tolperisone within 30 minutes, then absorbed and thereby quickly eliminated from the body. In the case of the sustained release oral medicinal product containing tolperisone according to the invention, the duration of dissolution of the active ingredient is longer, at least 80% of the active ingredient is released nearly evenly over the course of 6 hours, therefore, the desired therapeutic effect may be achieved by administering the product twice a day.

In order to ensure the stability of the sustained release tolperisone hydrochloride active ingredient following administration, it is necessary to establish an acidic micro-environment during dissolution, betaine hydrochloride is used for this in the present invention. It was empirically determined that the addition of an amount of betaine hydrochloride excipient at a ratio of 1:10-1:5 with respect to the amount of the tolperisone hydrochloride active ingredient does not influence the hardness of the product, nor does it have an effect on the speed of dissolution of the active ingredient either.

The hydrophilic matrix-forming material in the sustained release oral medicinal product containing tolperisone according to the invention serves, on the one part, as a binder for forming the granules and for ensuring the hardness of the tablet, and, on the other part, it forms a slowly dissolving/eroding gel in the aqueous medium and thereby slows down the dissolution of the active ingredient. Hydrophilic matrix-forming materials have a large molecular mass and their dissolution-slowing effect increases with the increase in molecular mass.

The sustained release oral medicinal product containing tolperisone according to the present invention is administered twice a day. In other words, the sustained release oral medicinal product containing tolperisone according to the invention is used at approximately 12-hour intervals - for example, one dose of the medicinal product in the morning and one in the evening - with which the even effect of the medicinal product may be ensured.

In addition to the tolperisone hydrochloride, the hydrophilic matrix-forming material and the betaine hydrochloride, the sustained release oral medicinal product containing tolperisone according to the invention may also contain a lubricant and an antiadhesion excipient. Lubricants are used in the case of the formulation of medicinal products if the friction occurring during the pressing of the pharmaceutical form (tablet) needs to be reduced. For a person skilled in pharmaceutical formulation, it is obvious that the use of too little lubricant can cause sticking, while too much lubricant may reduce the hardness of the tablet, and also have an influence on the speed of dissolution. Antiadhesion excipients improve the flow properties of powder mixtures and granulates, which, in the case of tabletting, results in the reduction of the weight distribution of the tablets.

In a preferable embodiment of the present invention, the sustained release oral medicinal product containing tolperisone contains 60 to 80 mass% tolperisone hydrochloride active ingredient with respect to the mass of the medicinal product. In addition to the active ingredient, the medicinal product also contains 5 to 15 mass% hydrophilic matrix-forming material, and 5 to 15 mass% betaine hydrochloride. Optionally, the sustained release oral medicinal product containing tolperisone according to the present invention, also contains 3 to 6 mass% lubricant, and 1 to 3 mass% antiadhesion excipient.

According to an even more preferable embodiment of the sustained release oral medicinal product containing tolperisone according to the present invention, it contains 60 to 70 mass% tolperisone hydrochloride, 10 to 15 mass% hydrophilic matrix-forming material, 10 to 15 mass% betaine hydrochloride, and optionally 4 to 5 mass% lubricant, and 1 to 2 mass% antiadhesion excipient calculated with respect to the mass of the medicinal product.

In other words, in the sustained release medicinal product according to the invention it is sufficient to use the hydrophilic matrix-forming material in an amount of 10 to 15 mass%, which is much less than the usual 30 to 50 mass% amount, or above in medicinal products administered once daily. As it may also be seen from the mass% composition and as stated above the amount of betaine hydrochloride excipient used in the medicinal product with respect to the amount of tolperisone hydrochloride active ingredient is usually in the ratio of between 1:10 and 1:5.

According to another preferable embodiment of the invention, the hydrophilic matrix-forming material is selected from the following list: hydroxypropyl cellulose (hypromellose), hydroxypropyl cellulose, polyethylene oxide, xanthan gum or polyacrylic acid. According to our experience, outstanding among the hydrophilic matrix-forming materials are polyethylene oxide and hypromellose, with respect to both the production of the tablet pharmaceutical form and the regulation of the speed of dissolution.

The speed of dissolution of the active ingredient of tablets, as we have already mentioned, is determined by the hydrophilic matrix-forming materials, therefore by their molecular mass and viscosity. During our experiments we found that polyethylene oxides with a viscosity above 4000 cP in a 2% aqueous solution and hypromelloses are appropriate in order to produce pharmaceutical forms displaying close to even dissolution over 6 hours. We found the Polyox WSR 303 type polyethylene oxide with a viscosity of 7500 to 10000 cP in a 1% aqueous solution to be particularly suitable, while the most suitable proved to be hypromellose with a nominal viscosity of 100000 cP in a 2% aqueous solution.

According to the prescription of the United States Pharmacopeia, the viscosity of such hypromellose measured using a Brookfield rotational viscometer may be 75% to 140% of the nominal value.

In a way surprising even for a person skilled in the art, we found that 5 to 15 mass% of hypromellose matrix-forming material with a nominal viscosity of 100000 cP in a 2% aqueous solution, is sufficient to appropriately slow down the speed of the dissolution of the active ingredient compared to the usual 30 to 50% or greater amount in retard medicinal products. With the use of less excipient, it is possible to produce pharmaceutical forms with a higher active ingredient content. Thereby it becomes possible to also produce pharmaceutical forms with an active ingredient content even above 60%, which, for example, by reducing the size of the tablet pharmaceutical form, makes it easier for the patient to swallow. This relatively small amount of hydrophilic matrix-forming excipient (5 to 15 mass%) is able to ensure the same rate of dissolution of the tolperisone active ingredient within the physiological pH range (between pH value 1 to 7) of the digestive system. In other words, the rate of dissolution of the tolperisone active ingredient is independent of the rate at which the medicinal product passes through the gastrointestinal system.

According to another preferable embodiment of the present invention, the lubricant is selected from the following list: stearic acid, stearic acid salts, glycerine stearates. Among the lubricants stearic acid is preferably used, as due to its acidic character it contributes to the stability of the tolperisone hydrochloride.

According to another preferable embodiment of the invention the antiadhesion excipient is talc or silicon dioxide; more preferably talc. Talc is more preferably used from among the antiadhesion excipients as it also has a lubricant effect, therefore it contributes to overcoming the sticking occurring during tabletting.

According to a particularly preferable embodiment of the present invention, the sustained release oral medicinal product containing tolperisone consists of tolperisone hydrochloride, hydrophilic matrix-forming material, betaine hydrochloride, talc and stearic acid. It is obvious for a person skilled in the art that all solid pharmaceutical forms contain some water, but in practice, here, the water is not a component, it is the moisture content of the medicinal product.

The sustained release oral medicinal product containing tolperisone according to the present invention is in the form of a tablet.

The sustained release oral medicinal product containing tolperisone according to the invention contains 200 to 400 mg tolperisone hydrochloride, more preferably 225 to 300 mg tolperisone hydrochloride. On the basis of our measurements the pharmaceutical form containing tolperisone hydrochloride that contains the active ingredient in a dose of 200 to 400 mg is able to release at least 80% of the active ingredient within 6 hours measured with a dissolution test using the paddle method.

The method for producing the sustained release oral medicinal product containing tolperisone contains the following steps:
a) all of the tolperisone hydrochloride, all of the betaine hydrochloride and at least 30% of the hydrophilic matrix-forming material or all of it are homogenised, then granulated with the addition of water;
b) the granulate obtained in step a) is dried;
c) the dry granulate is sieved;
d) the granulate according to step c) is homogenised with the lubricant, the antiadhesion excipient and, optionally, with the remainder of the hydrophilic matrix-forming material;
e) the homogenised material obtained from step d) is pressed into tablets;
f) the tablets according to step e) are optionally provided with a film coating.

In step a) the tolperisone hydrochloride, the betaine hydrochloride and all of the hydrophilic matrix-forming material, or at least 30% of it, is homogenised and moistened with water. The homogenate needs to be moistened because in this way the liquid bridges created as a result of the adhesion of the granules are transformed into solid material bridges after the wetting agent is removed, thereby larger particles, granules are created, which have better flow and better compressing properties. In other words, step a) is actually a wet granulation step.

The granulate obtained in step a) is dried in a fluidisation apparatus to a moisture content of 1%.

Following this the dry granulate obtained in step b) is passed through a 1.5 mm sieve.

In step d) the granulate sieved to size according to step c) is homogenised with the lubricant, the antiadhesion excipient and, optionally, the remainder of the hydrophilic matrix-forming material (if only at least 30% of the hydrophilic matrix-forming material was used in step a) for the production of the granulate).

In step e) the homogenised material according to step d) is pressed into tablets.

In step f) the tablets are optionally provided with a film coating.

The use of the sustained release oral medicinal product containing tolperisone in a method for the treatment of symptoms related to spastic paralysis, or for the reduction of painful muscle spasms, or for the improvement of muscle tone, where the form of the medicinal product is preferably a tablet.

The use of the sustained release oral medicinal product containing tolperisone according to the above also relates to the use in a method for the treatment of symptoms related to spastic paralysis, or for the reduction of painful muscle spasms, or for the improvement of muscle tone, in the case of which the medicinal product is administered twice a day to the patient.

### Examples:

Further details of the invention are presented in the following examples without limiting the invention to the examples.

The quality testing of the tablets was performed on the basis of the prescriptions of the European Pharmacopoeia (Ph. Eur.), which are equal to those of the Japanese pharmacopoeia (JP), and of the United States Pharmacopeia (USP).

The hardness of the tablets was tested using the apparatus and method prescribed in the European Pharmacopoeia chapter *2.9.8. Resistance to crushing of tablets.* The force required to crush the tablets is given in N. The tests were performed using a Kraemer UTS 4.1 automatic apparatus.

The testing of the active ingredient release of the tablets was performed by paddle method and method according to the prescriptions of the European Pharmacopoeia chapter *2.9.3. Dissolution test for solid dosage forms.* A total of 900 ml 37 ± 0.5 °C, 0.1 N hydrochloric acid solution was used as dissolution medium and a pH 4.5, and a pH 6.8 buffer was used, the mixing speed was 50 revolutions/minute. The amount of active ingredient released from the tablets was given as a percentage of the nominal active ingredient content of the tablets. The tests were performed using an Erweka automatic tablet dissolution tester apparatus. The results are given as the average of the results measured after the given mixing (dissolution) time of six tablets.

### Example 1: Direct tabletting, briquetting (for comparison)

225 g tolperisone hydrochloride and 56.25 g and 56.25 g hydrophilic matrix-forming material, namely a mixture of Polyox WSR 303 polyethylene oxide, Methocel K100M hypromellose, and Carbopol 71G carbomer were placed separately into the material container of a Procept 4M8 eddy current granulation device. The materials were homogenised for 10 minutes, then after 0.75 g and 0.75 g magnesium stearate were separately added it was homogenised for a further 2 minutes. The homogenised mixture was pressed using a Korsch EX0 tablet press machine using an 8-mm biconvex pressing tool to produce tablets with an average weight of 282 mg.

According to the experience gained the quality of the tablets produced in this way was not satisfactory, they displayed flaking and surface adhesion.

After briquetting the homogenised mixture (pressing with great pressure and grinding) and the addition of an additional 1% magnesium stearate the compressibility could not be improved.

### Example 2: the production of a sustained release oral medicinal product containing tolperisone

Medicinal products with the following composition were produced (table 1), where tolperisone HCl means tolperisone hydrochloride, betaine HCl means betaine hydrochloride, among the hydrophilic matrix-forming materials responsible for extended dissolution we tried the Methocel K100M type hypromellose / the Polyox WSR 303 type polyethylene oxide, purified water was used for the wet granulation. In the case of these compositions we chose talc as the antiadhesion excipient, and stearic acid as the lubricant. In table 1 the values given are expressed in grams.

**Table 1**

| Component (g) | 1/A | 1/B | 1/C | 1/D |
|---|---|---|---|---|
| Tolperisone HCl | 225 | 225 | 225 | 225 |
| Betaine HCl | 45 | 45 | 45 | 45 |
| Methocel K 100M | 15 | - | 30 | 30 |
| Polyox WSR 303 | - | 15 | - | - |
| Purified water | 40 | 40 | 130 | 130 |

| External phase | | | | |
|---|---|---|---|---|
| Polyox WSR 303 | - | 50 | 30 | |
| Methocel K 100M | 50 | - | - | 30 |
| Talc | 5 | 5 | 5 | 5 |
| Stearic acid | 15 | 15 | 15 | 15 |
| Remark | Could not be tabletted | Could not be tabletted | Tabletizable | Tabletizable |

225 g tolperisone hydrochloride, 45 g betaine hydrochloride and the given amount (see table 1) of hydrophilic matrix-forming material (namely the Polyox WSR 303 type polyethylene oxide or the Methocel K100M type hypromellose) were placed separately in a Procept 4M8 eddy current granulation device. The materials were homogenised for 3 minutes, then granulated for 5 minutes with the given amount of water (40 or 130 g). The wet granulate was dried to a moisture content of approx. 1% in a fluidisation apparatus, then passed through a 1.5-mm sieve.

The sieved-to-size granulate was homogenised with the materials of the external phase, i.e. with the other specified part of the hydrophilic matrix-forming material, talc and stearic acid.

The homogenate was pressed in a Korsch EX0 tablet press using a 10-mm biconvex press tool to make tablets with an average weight of 350 mg and a hardness of 80-100 N.

According to the experience obtained the quality of the tablets proved to be satisfactory in the case of the use of approx. 10% Methocel K100M type hypromellose (hydrophilic matrix-forming material) during granulation (see production batches 1/C and 1/D).

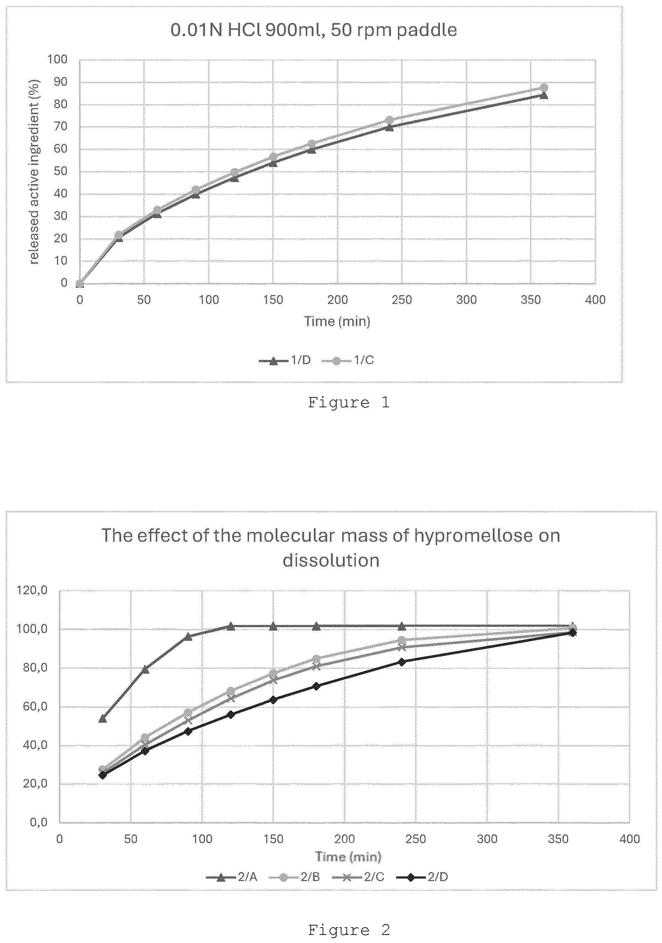

Figure 1 shows the in vitro dissolution data of the tablets originating from production batches 1/C and 1/D.

As it may be clearly seen in figure 1 in the case of production batches 1/C and 1/D, the dissolution of the active ingredient from the tablets takes place at a nearly even rate over the course of 6 hours. The degree of dissolution of the active ingredient content over 6 hours exceeds 80% in both cases.

### Example 3: the testing of the effect of hypromelloses with different molecular masses

The molecular mass of the hypromelloses is characterised by the viscosity in 2% aqueous solution.

The viscosity of the 100 LV type hypromellose is approximately 100 cP, and that of the 4M, 15M, and 100M type hypromelloses is respectively approximately 4000, 10000, and 100000 cP. According to the prescription of the pharmacopoeia the deviation of the measured viscosity value of the hypromellose from the nominal value given for the hypromellose may be ±20%, or between 75% and 140% depending on the measurement method.

The compositions of the products according to table 2 (2/A to 2/D) differed in terms of the molecular mass of the hypromelloses. In table 2 the values given are expressed in grams.

**Table 2**

| Component | 2/A | 2/B | 2/C | 2/D |
|---|---|---|---|---|
| Tolperisone HCl | 225 | 225 | 225 | 225 |
| Betaine HCl | 22.5 | 22.5 | 22.5 | 22.5 |
| Methocel K 100LV | 30 | - | - | - |
| Methocel K 4M | - | 30 | - | - |
| Methocel K 4M | - | - | 30 | - |
| Methocel K 100M | - | - | - | 30 |
| Purified water | 50 | 50 | 50 | 50 |
| Talc | 5 | 5 | 5 | 5 |
| Stearic acid | 15 | 15 | 15 | 15 |
| Remark | Good tablets can be produced | Good tablets can be produced | Tabletizable | Tabletizable |

The tablets were produced in accordance with the method in example 2 with the difference that tablets with a weight of 297.5 mg and a hardness of 80-100 N were pressed with a 9-mm biconvex press tool.

The press quality of the homogenate proved to be satisfactory in all cases.

As it may be seen from the dissolution data in figure 2, hypromelloses with a viscosity of at least 4000 cP in a 2% aqueous solution (i.e. production batches 2/B, 2/C and 2/D) are suitable for the appropriate slowing of the dissolution speed of the tolperisone hydrochloride. By increasing the molecular mass (viscosity) the amount of active ingredient released over the course of 8 hours does not change, nearly 100%, however the rate of dissolution becomes more uneven. The compositions of the products in experiments 2/B and 2/C are the same. The large degree of agreement between the dissolution values verifies the reproducibility of the production technology.

### Example 4: testing of the pH-dependence of dissolution

With respect to the fact that the pH of the digestive medium in the human gastrointestinal system varies between 1 and 7 pH, it is important that the active ingredient release of sustained release products displays only minimal fluctuation in this pH range.

The testing of this was performed in the case of the products with the compositions as follows.

The composition of the product is summarised in table 3, where the values are given in grams.

**Table 3**

| Component | Sample 3 |
|---|---|
| Tolperisone HCl | 225 |
| Betaine HCl | 45 |
| Methocel K 100M | 30 |
| Purified water | 65 |
| Methocel K 100M | 15 |
| Talc | 5 |
| Stearic acid | 15 |
| Remark | Tabletizable |

The tablets were produced using the method according to example 2, with the difference that a 10-mm diameter biconvex press tool was used to produce tablets with a weight of 335 mg, and a hardness of 100-120 N.

The dissolution of the tablets was measured over the course of 6 hours in aqueous buffer solutions at pH values of pH 1.0; pH 4.5, and pH 6.8.

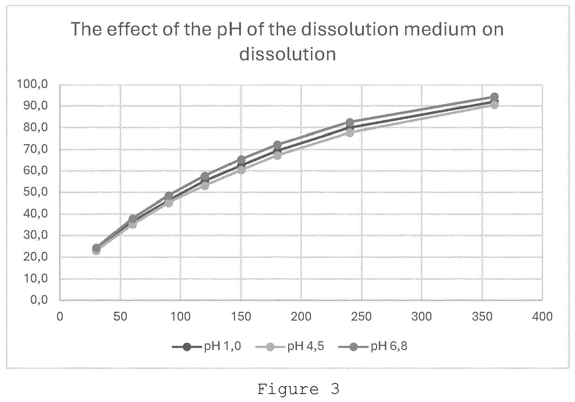

Figure 3 illustrates the average of the dissolution results of six tablets.

On the basis of the data the pH of the dissolution medium does not influence the rate of dissolution, therefore the product is suitable for the even release of active ingredient in the gastrointestinal system irrespective of the rate of passage of the product.

## Claims

1. Sustained release oral medicinal product containing tolperisone, **characterised by that** it contains tolperisone hydrochloride as active ingredient, a hydrophilic matrix-forming material and betaine hydrochloride as excipient, and which medicinal product releases at least 80% of the tolperisone hydrochloride active ingredient within 6 hours measured using a dissolution test performed by paddle method.

2. Sustained release oral medicinal product containing tolperisone according to claim 1, **that** contains a lubricant and an antiadhesion excipient.

3. Sustained release oral medicinal product containing tolperisone according to any of claims 1 to 2, **that** contains 60 to 80 mass% tolperisone hydrochloride, 5 to 15 mass% hydrophilic matrix-forming material, 5 to 15 mass% betaine hydrochloride and optionally 3 to 6 mass% lubricant, and 1 to 3 mass% antiadhesion excipient with respect to the mass of the medicinal product.

4. Sustained release oral medicinal product containing tolperisone according to any of claims 1 to 3, **that** even more preferably contains 60 to 70 mass% tolperisone hydrochloride, 10 to 15 mass% hydrophilic matrix-forming material, 10 to 15 mass% betaine hydrochloride and optionally 4 to 5 mass% lubricant, and 1 to 2 mass% antiadhesion excipient with respect to the mass of the medicinal product.

5. Sustained release oral medicinal product containing tolperisone according to any of claims 1 to 4, **wherein** the hydrophilic matrix-forming material is selected from the following list: hydroxypropyl methylcellulose (hypromellose), hydroxypropyl cellulose, polyethylene oxide, xanthan gum or polyacrylic acid; the hydrophilic matrix-forming material is more preferably hypromellose.

6. Sustained release oral medicinal product containing tolperisone according to any of claims 1 to 5, **wherein** the viscosity of the hydrophilic matrix-forming material in a 2% aqueous solution, is over 4000 centipoise (cP), more preferably nominally 100000 cP.

7. Sustained release oral medicinal product containing tolperisone according to any of claims 1 to 6, **wherein** the lubricant is selected from the following list: stearic acid, stearic acid salts, glycerine stearates; the lubricant is more preferably stearic acid.

8. Sustained release oral medicinal product containing tolperisone according to any of claims 1 to 7, **wherein** the antiadhesion excipient is talc or silicon dioxide; more preferably talc.

9. Sustained release oral medicinal product containing tolperisone according to any of claims 1 to 8, **that** consists of tolperisone hydrochloride, hydrophilic matrix-forming material, betaine hydrochloride, talc and stearic acid.

10. Sustained release oral medicinal product containing tolperisone according to any of claims 1 to 9, the pharmaceutical form **of which** is a tablet.

11. Sustained release oral medicinal product containing tolperisone according to any of claims 1 to 10, **that** contains 200 to 400 mg tolperisone hydrochloride, more preferably 225 to 300 mg tolperisone hydrochloride.

12. Method for the production of the sustained release oral medicinal product containing tolperisone according to any of claims 1 to 11, **characterised by that** the method contains the following steps:
a) all of the tolperisone hydrochloride, all of the betaine hydrochloride and at least 30% of the hydrophilic matrix-forming material or all of it are homogenised, then granulated with the addition of water;
b) the granulate obtained in step a) is dried;
c) the dry granulate is sieved;
d) the granulate according to step c) is homogenised with the lubricant, the antiadhesion excipient and, optionally, with the remainder of the hydrophilic matrix-forming material;
e) the homogenised material obtained from step d) is pressed into tablets;
f) the tablets according to step e) are optionally provided with a film coating.

13. The use of the sustained release oral medicinal product containing tolperisone according to any of claims 1 to 11 for the treatment of symptoms related to spastic paralysis, or for the reduction of painful muscle spasms, or in a method for the improvement of muscle tone, **characterised by that** the form of the medicinal product is a tablet.

14. The use of the sustained release oral medicinal product containing tolperisone according to any of claims 1 to 11 for the treatment of symptoms related to spastic paralysis, or for the reduction of painful muscle spasms, or in a method for the improvement of muscle tone, **characterised by** that the medicinal product is administered to the patients twice a day.
